(19) Europäisches Patentamt
European Patent Office
Office européen des brevets

(11) EP 0 773 024 A2

(12) EUROPEAN PATENT APPLICATION

(43) Date of publication:
**14.05.1997 Bulletin 1997/20**

(51) Int Cl.[6]: **A61K 31/44**, A61K 31/445

(21) Application number: **96307171.7**

(22) Date of filing: **01.10.1996**

(84) Designated Contracting States:
**AT BE CH DE DK ES FI FR GB GR IE IT LI LU NL PT SE**

(30) Priority: **27.10.1995 US 8024**

(71) Applicant: **PFIZER INC.**
**New York, N.Y. 10017 (US)**

(72) Inventors:
- **Cheng, John B.**
  **Waterford, Connecticut 06385 (US)**
- **Marfat, Anthony**
  **Mystic, Connecticut 06355 (US)**
- **Watson, John W.**
  **Ledyard, Connecticut 06339 (US)**

(74) Representative: **Moore, James William**
**Pfizer Limited,**
**European Patent Department,**
**Ramsgate Road**
**Sandwich, Kent CT13 9NJ (GB)**

# (54) Use of N-substituted nicotinamide compounds for the treatment of acute and chronic inflammatory diseases

(57) The use of a compound of the formula I

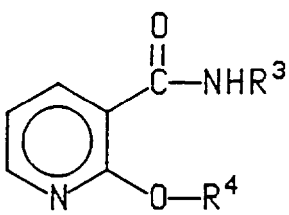

I ,

or a pharmaceutically acceptable acid addition salt thereof, wherein

$R^3$ is 1-piperidyl, 1-(3-indolyl)ethyl, ($C_1$-$C_4$)-alkyl, phenyl, benzyl, 1-(1-phenylethyl) or monosubstituted benzyl wherein the substituent is chloro, fluoro, methyl or methoxy and said substituent is on the aromatic ring;
$R^4$ is bicyclo[2.2.1]hept-2-yl or a group of the formula II

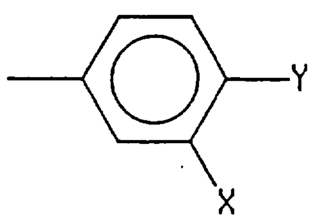

II

wherein Y is hydrogen, fluoro or chloro; and
X is hydrogen, fluoro, chloro, methoxy, trifluoromethyl, cyano, carboxy, methylcarbamoyl, dimethyl-carbamoyl or carbo($C_1$-$C_4$)alkoxy;

EP 0 773 024 A2

for the manufacture of a medicament for inhibiting phosphodiesterase (PDE) type IV or the production of tumor necrosis factor (TNF).

## Description

This invention relates to the use of certain N-substituted nicotinamide compounds and their pharmaceutically acceptable acid addition salts for the treatment of acute and chronic inflammatory disease in mammals, including humans.

N-3-(4-methoxybenzyl)-2-[4-fluorophenoxy]nicotinamide having the structural formula

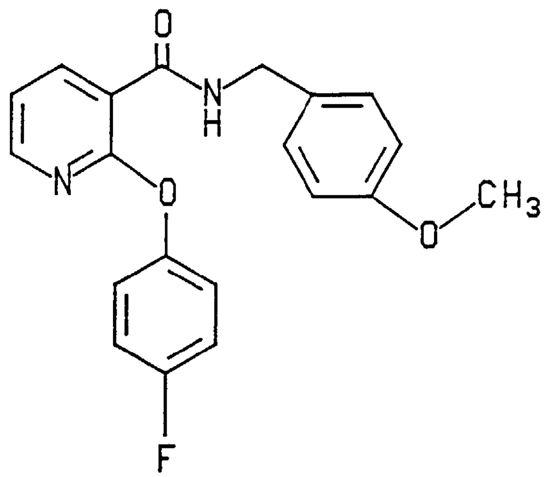

and other N-substituted nicotinamide compounds are referred to in United States Patent 4,861,891 which issued on August 29, 1989. This patent refers to the use of such compounds as selective inhibitors of type IV phosphodiesterase (PDE IV). This patent is incorporated herein by reference in its entirety.

The N-substituted nicotinamide compounds referred to below as the "compounds of formula I", which includes N-3-methoxybenzyl-2-phenoxynicotinamide, are also useful in potentiation of $PGE_1$ - induced cAMP elevation in U-937 cells, inhibition of leukotriene synthesis and mediator release in human eosinophils. These compounds also exhibit reduced emetic response in ferrets.

This invention relates to a method of inhibiting phosphodiesterase type IV or the production of tumor necrosis factor in mammals, particularly humans, comprising administering to said mammal an anti-inflammatory amount of a compound of the formula

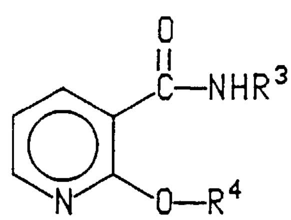

I

or a pharmaceutically acceptable acid addition salt thereof wherein

$R^3$ is 1-piperidyl, 1-(3-indolyl)ethyl, $(C_1$-$C_4)$-alkyl, phenyl, benzyl, 1-(1-phenylethyl) or monosubstituted benzyl wherein the substituent is chloro, fluoro, methyl or methoxy and said substituent is on the aromatic ring;

$R^4$ is bicyclo[2.2.1]hept-2-yl or a group of the formula

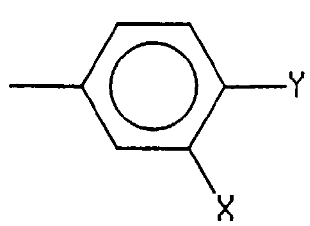

II

wherein Y is hydrogen, fluoro or chloro; and

X is hydrogen, fluoro, chloro, methoxy, trifluoromethyl, cyano, carboxy, methylcarbamoyl, dimethyl-carbamoyl or carbo($C_1$-$C_4$)alkoxy.

A preferred embodiment of this invention relates to the above method of treating inflammatory diseases wherein the compound administered is one wherein $R^3$ is 1-piperidyl or 1-(3-indolyl)ethyl.

Another preferred embodiment of this invention relates to the foregoing preferred embodiment wherein, in the compound that is administered, $R^3$ is 1-(3-indolyl)ethyl and $R^4$ is bicyclo[2.2.1]hept-2-yl.

Another preferred embodiment of this invention relates to the foregoing preferred embodiment wherein, in the compound that is administered, $R^4$ is

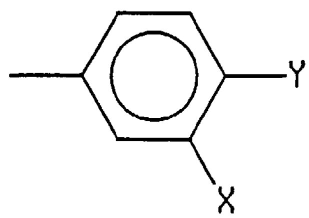

II

wherein Y is hydrogen and X is carbo($C_1$-$C_4$)alkoxy.

Another preferred embodiment of this invention relates to the foregoing preferred embodiment wherein, in the compound that is administered, $R^3$ is 1-piperidyl and $R^4$ is

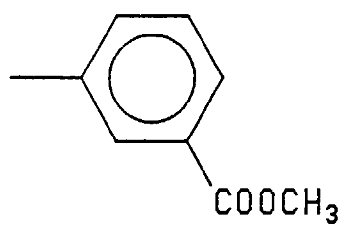

III

.

Another preferred embodiment of this invention relates to the above method of treating or preventing a condition selected from the group consisting of asthma, chronic bronchitis, atopic dermatitis, urticaria, allergic rhinitis, allergic conjunctivitis, vemal conjunctivitis, cosinophilic granuloma, psoriasis, rheumatoid arthritis, septic shock, ulcerative colitis, Crohn's disease reperfusion injury of the myocardium and brain, chronic glomerulonephritis, endotoxic shock, adult respiratory distress syndrome, diabetes insipidus, multiple sclerosis and central nervous system disorders, as well as other diseases involving the production of TNF, comprising administering to a patient an effective amount of a compound of formula

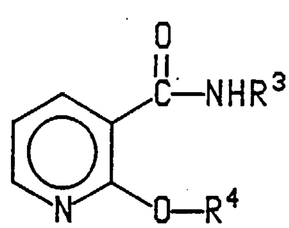

I

or a pharmaceutically acceptable acid addition salt thereof wherein

$R^3$ is 1-piperidyl, 1-(3-indolyl)ethyl, ($C_1$-$C_4$)-alkyl, phenyl, benzyl, 1-(1-phenylethyl) or monosubstituted benzyl wherein the substituent is chloro, fluoro, methyl or methoxy and said substituent is on the aromatic ring;

$R^4$ is bicyclo[2.2.1]hept-2-yl or a group of the formula

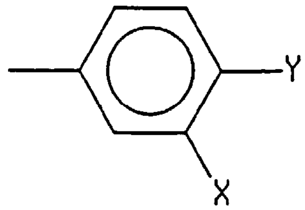

II

wherein Y is hydrogen, fluoro or chloro; and
X is hydrogen, fluoro, chloro, methoxy, trifluoromethyl, cyano, carboxy, methylcarbamoyl, dimethyl-carbamoyl or carbo($C_1$-$C_4$)alkoxy.

Compounds of formula **I** and their pharmaceutically acceptable acid addition salts may be prepared as described in United States Patent 4,861,891 referred to above.

Phosphodiesterase IV inhibitors are useful in the treatment of a variety of allergic and inflammatory diseases including: asthma, chronic bronchitis, atopic dermatitis, urticaria, allergic rhinitis, allergic conjunctivitis, vemal conjunctivitis, cosinophilic granuloma, psoriasis, rheumatoid arthritis, septic shock, ulcerative colitis, Crohn's disease reperfusion injury of the myocardium and brain, chronic glomerulonephritis, endotoxic shock and adult respiratory distress syndrome. In addition, PDE IV inhibitors are useful in the treatment of diabetes insipidus, multiple sclerosis and central nervous system disorders such as depression and multi-infarct dementia.

Phosphodiesterase type IV inhibitors have been shown to inhibit tumor necrosis factor production. The compounds of the formula **I** are also useful in the treatment of viral infections, where such viruses are sensitive to upregulation by TNF or will elicit TNF production in vivo. The viruses contemplated for treatment herein are those that produce TNF as a result of infection, or those which are sensitive to inhibition, such as by decreased replication, directly or indirectly, by the TNF inhibitors of the formula **I**. Such viruses include, but are not limited to HIV-1, HIV-2 and HIV-3, cytomegalovirus (CMV), influenza, adenovirus and the Herpes group of viruses, such as, but not limited to, Herpes zoster and Herpes simplex.

This invention more specifically relates to a method of treating a mammal, afflicted with a human immunodeficiency virus (HIV), which comprises administering to such mammal an effective TNF inhibiting amount of a compound of the formula **I**.

The compounds of the formula **I** may also be used in association with the veterinary treatment of animals, other than in humans, in need of inhibition of TNF production. TNF mediated diseases for treatment, therapeutically or prophylactically, in animals include disease states such as those noted above, but in particular viral infections. Examples of such viruses include, but are not limited to feline immunodeficiency virus (FIV) or other retroviral infection such as equine infectious anemia virus, caprine arthritis virus, visna virus, maedi virus and other lentiviruses.

The compounds of the formula **I** are also useful in the treatment of yeast and fungal infections, where such yeast and fungi are sensitive to upregulation by TNF or will elicit TNF production in vivo. A preferred disease state for treatment is fungal meningitis. Additionally, the compounds of the formula **I** may be administered in conjunction with other drugs of choice for systemic yeast and fungal infections. Drugs of choice for fungal infections, include but are not limited to the class of compounds called the polymixins, such as Polymycin B, the class of compounds called the imidazoles, such as clotrimazole, econazole, miconazole, and ketoconazole; the class of compounds called the triazoles, such as fluconazole, and itranazole, and the class of compound called the Amphotericins, in particular Amphotericin B and liposomal Amphotericin B.

The co-administration of the anti-fungal agent with a compound of the formula **I** may be in any preferred composition for that compound such as is well known to those skilled in the art, for instance the various Amphotericin B formulations. Co-administration of an anti-fungal agent with a compound of the formula **I** may mean simultaneous administration or in practice, separate administration of the agents to the mammal but in a consecutive manner. In particular, the compounds of the formula **I** may be co-administered with a formulation of Amphotericin B, notably for systemic fungal infections. The preferred organism for treatment is the Candida organism. The compounds of the formula **I** may be co-administered in a similar manner with anti-viral or anti-bacterial agents.

The compounds of the formula **I** may also be used for inhibiting and/or reducing the toxicity of an anti-fungal, anti-bacterial or anti-viral agent by administering an effective amount of a compound of the formula **I** to a mammal in need of such treatment. Preferably, a compound of the formula **I** is administered for inhibiting or reducing the toxicity of the Amphotericin class of compounds, in particular Amphotericin B.

The methods of this invention comprise administering compounds of the formula **I** and their pharmaceutically acceptable acid salts and solvates of such compounds and salts (hereinafter collectively referred to as "the therapeutic agents") to a mammal. The therapeutic agents can be administered to said mammal either alone or, preferably, in combination with pharmaceutically acceptable carriers or diluents in a pharmaceutical composition, according to standard pharmaceutical practice. Such administration can be accomplished via a variety of routes, including oral, parenteral and topical. Parenteral administration, as used herein, includes but is not limited to intravenous, intramuscular, intraperitoneal, subcutaneous, and transdermal administration. It is generally preferred to administer the therapeutic agents orally.

For use in the treatment of acute and chronic inflammatory diseases, the therapeutic agents are most desirably administered, in accordance with this invention, in doses ranging from about 5 mg to about 250 mg per day, preferably from about 20 mg to about 120 mg per day, in single or divided doses. For intranasal or inhaler administration, the dosage is generally formulated as a 0.1 to 1% (w/v) solution. In practice the physician will determine the actual dosage which will be most suitable for an individual patient and it will vary with the age, weight and response of the particular patient. The above dosages are exemplary of the average case but there can, of course, be individual instances where higher or lower dosage ranges are merited, and all such dosages are within the scope of this invention.

In some instances, dosage levels below the lower limit of the above dosage ranges may be more than adequate, while in other cases still larger dosages may be employed without causing any harmful or deleterious side effects to occur, provided that such higher dosage levels are first divided into several smaller doses that are to be administered throughout the day.

For purposes of oral administration, tablets containing excipients such as sodium citrate, calcium carbonate and dicalcium phosphate may be employed along with various disintegrants such as starch and preferably potato or tapioca starch, alginic acid and certain complex silicates, together with binding agents such as polyvinylpyrrolidone, sucrose, gelatin and acacia. Additionally, lubricating agents such as, but not limited to, magnesium stearate, sodium lauryl sulfate and talc are often very useful for tableting purposes. Solid compositions of a similar type may also be employed as fillers in soft elastic and hard filled gelatin capsules. Preferred materials in this connection also include, by way of example and not of limitation, lactose or milk sugar as well as high molecular weight polyethylene glycols. When aqueous suspensions and/or elixirs are desired for oral administration, the essential active ingredient may be combined with various sweetening or flavoring agents, coloring matter or dyes and, if so desired, emulsifying and/or suspending agents, together with diluents such as water, ethanol, propylene glycol, glycerine and various like combinations thereof.

For purposes of parenteral administration, solutions of a therapeutic agent in sesame or peanut oil or in aqueous propylene glycol may be employed, as well as sterile aqueous solutions of the corresponding water soluble base salts previously enumerated. Such aqueous solutions should be suitably buffered if necessary, and the liquid diluent rendered isotonic with sufficient saline or glucose. These particular aqueous solutions are especially suitable for intravenous, intramuscular and subcutaneous injection purposes. In this connection, the sterile aqueous media employed are readily obtained by standard techniques well known to those skilled in the art. For instance, distilled water is ordinarily used as the liquid diluent and the final preparation is passed through a suitable bacterial filter such as a sintered glass filter or a diatomaceous-earth or unglazed porcelain filter. Preferred filters of this type include the Berkefeld, the Chamberland and the Asbestos Disk-Metal Seitz filter, wherein the fluid is sucked into a sterile container with the aid of a suction pump. The necessary steps should be taken throughout the preparation of these injection solutions to insure that the final products are obtained in a sterile condition.

For purposes of transdermal administration, the dosage form of a particular therapeutic agent may include, by way of example, solutions, lotions, ointments, creams, gels, suppositories, rate limiting sustained release formulations and devices. Such dosage forms comprise the particular compound and may include ethanol, water, penetration enhancers and inert carriers such as gel producing materials, mineral oil, emulsifying agents, benzyl alcohol and the like. Specifictransdermal flux enhancing compositions are disclosed in European Patent Applications 271,983 and 331,382, which were published, respectively, on June 22, 1988 and September 6, 1989. These applications are incorporated herein by reference in their entireties.

The ability of the compounds of formula I to inhibit PDE IV and exhibit reduced emetic activity may be determined, respectively, as described in the following protocols.

Inhibition of Type IV Phosphodiesterase (PDEIV)

Human recombinant PDEIVs were expressed in a Baculovirus/SF-9 cell system (Pollok B, Fuog E, Robbins M, Fisher D, Umland J. Pillar J, and Cheng J: Baculovirus expression of human phosphodiesterase (PDE) IV isoenzymes: comparison with rPDE-IV produced in mammalian and bacterial expression system. Baculovirus and Insect Cell Gene Expression Conference, Pinehurst, N.C., March 26-30, 1995). The SF-9 cell lysate was capable of hydrolyzing cyclic AMP (cAMP) and had no effect on cyclic GMP (cAMP). Kinetic analyses of these enzymes indicated that the Michaelis constant, Km, to hydrolize cAMP ranged from 0.5-4 μM. The enzyme was sensitive to magnesium chloride, and was inhibited by the standard PDEIV inhibitor, rolipram. These preparations were used to evaluate the ability of test compounds for inhibition of PDEIV.

Preparation of test compounds: Compounds were dissolved in methyl sulfoxide at a concentration of $10^{-2}$M, then diluted 1:25 in water (4 x $10^{-4}$M compound, 4% methyl sulfoxide). Further serial dilutions are made in 4% methyl

sulfoxide to achieve desired concentrations. Final methyl sulfoxide concentration in assay tubes was 1%.

In triplicate, the following were added to a 12 x 75 mm glass tube, in order, at 4°C: (all concentrations are given as final concentrations in assay tube)

25 μl compound or methyl sulfoxide (1%, for control and blank)
25 μl assay buffer (50 mM Tris, 10mM magnesium chloride, pH 7.5)
25 μl [3H]-cAMP (1 μM)
25 μl PDEIV enzyme (for blank, enzyme is preincubated in boiling water bath for 10 minutes.

The reaction tubes were shaken and placed in a water bath (37°C) for 10 to 30 minutes, at which time the reaction was stopped by placing the tubes in a boiling water bath for 2 minutes. Washing buffer (0.5 ml, 0.1M HEPES/0.1M sodium chloride, pH 8.5) was added to each tube in an ice bath. The contents of each tube were applied to an Affi-Gel 601 column (boronate affinity gel, 1.2 mL bed volume) previously equilibrated with washing buffer. [3H]cAMP was washed with 2 x 6 mL washing buffer, and [3H]5'AMP was then eluted with 6 mL 0.25M acetic acid. After vortexing, 1 mL of the elution was added to 3 mL Atomlight scintillation fluid in an appropriate vial, votexed, and counted for [$^3$H].

Percent inhibition is determined by the formula:

$$\%inh = \left[1 - \frac{\text{avg. cpm (test compound)} - \text{avg. cpm (blank (boiled enzyme))}}{\text{avg. cpm (control (no compound)} - \text{avg. cpm (blank (boiled enzyme))}}\right] \times 100\%$$

$IC_{50}$ is defined as that concentration of compound which inhibits 50% of hydrolysis of [3H]cAMP to [3H]5'AMP.

On some experiment, test compounds were evaluated for PDEIV inhibition by using one-step enzyme assay that was similar to the method reported by Thompson et al. (Thompson JW, Brokker G, and Appleman MM: Assay of cyclic nucleotide phosphodiesterases with radioactive substrates. Methods in Enzymology 38: 205-212, 1974).

Evaluation of Cyclic AMP (cAMP) Elevation of Compounds

Human U937 cells grown in continuous culture were obtained and spun at 1400 rpm in a Sorvall RT6000B at 22°C for 5 minutes. The supematant was decanted, and the cell pellet was resuspended in RPMI 1640 cell culture medium plus 2% fetal bovine serum (hereinafter FBS). Cells were counted and viability was checked using a hemocytomster. An appropriate volume of RPMI + 2% FBS was added to the cell suspension to make the cell concentration $10^6$ cells/ml. Five hundred μl ($5X10^5$ cells) of the suspension was added to a 12X75 mm glass tube 5μl containing +/- test compound, in duplicate or triplicate. This mixture was allowed to incubate at 37°C for 15 minutes. One μl PGE1 was added and the incubation was allowed to proceed for another 15 minutes, at which time the tubes were placed in a boiling water bath for 10 minutes.

Tubes were spun for 10 minutes at 3700 rpm in the Sorvall RT6000B. Ten μl of supematant were carefully sampled for radioimmunoassay (hereinafter RIA). A cAMP RIA kit (new England Nuclear cat no. NEK-033) was used to quantitate cAMP in each tube. Directions were followed according to the procedure for a non-acetylated assay as described in the RIA manual provided with the kit.

cAMP values were corrected for dilutions and cell concentration and expressed as picomoles (pmol) cAMP produced per $1X10^6$ cells. Mean value for each sample +/- standard error (if applicable) were also calculated. Either of two measurements of compound potency (EC50 or PC50) were calculated. EC50 is that concentration of compound which produces 50% of the arbitrary maximal response (e.g., that concentration of cAMP produced by 10μM rolipram) after substraction of a baseline response. PC50 is that concentration of compound which produces 50% of the maximal response of that compound after substraction of the baseline response. The maximal response of a test compound must be at least 150% of the baseline response in order for the PC50 to be calculated.

Inhibition of EDN/$LTE_4$ Production of Compounds

One hundred ml blood was obtained from healthy donors in Vacutainer tube #6480 (143 USP Units Sodium Heparin, 10ml draw). Heparinized whole blood was pooled in a siliconized glass beaker or 50ml conical centrifuge tubes at room temperature. A whole blood smear was performed using the Diff-Quik method to determine the percent eosinophils relative to total mononuclear leukocytes. One ml whole blood was palced in a 12X75mm polypropylene or siliconized glass tube containing 1µl methyl sulfoxide or 1µl 1000X test compound in triplicate. After vortexing, tubes were placed in a shaking water bath at 37°C for 15 minutes. One µl PGE1 in methyl sulfoxide was added to all tubes to give a final concentration of 1µm or 0.1µm PGE1 . After vortexing, 100µl PBS (negative control) or Sephadex G-15 beads in PBS (8.25 to 16.5 mg/ml final conc.) was added to tubes. After vortexing, all tubes were incubated in a shaking water bath at 37°C for 1 to 2 hours.

At the conclusion of the incubation, 20µl of 15% ethylenediaminetetraacetic acid in PBS was added to each assay tube to give a final concentration of 0.3%. After vortexing, all assay tubes were centrifuged at 2000 rpm at 22°C, for 5 minutes.

All plasma supematants were tested for EDN levels. EPX RIA was performed according to kit manufacturer's instructions (Kabi Pharmacia Diagnostics) with all volumes 5 times less than recommended (e.g., 10µl unknown sample volume). EDN and EPX have been shown to be identical proteins (Dahl et al., Asthma: Basic Mechanisms and Clinical Management 2nd ed. p112, 1992). EDN levels are calculated by comparison to a standard curve using Microsoft Excel or other appropriate software and are expressed as ng/ml. Percent of control EDN release is calculated by:

% control EDN = (EDN sample-EDN blank)/(EDN total-EDN blank)

where EDN blank is the EDN release in the absence of Sephadex G-15 and EDN total is the EDN release in the presence of Sephadex G-15. Where applicable, an IC30 is determined for test compounds using Microsoft Excel or other appropriate software.

LTE4 Enzyme Immunoassay (ETA)

All plasma supematants were tested for LTE4 levels. LTE4 EIA was performed according to kit manufacturer's instructions (Cayman Chemical) using a 1:10 plasma sample dilution in EIA buffer as unknown. LTE4 standards other than that provided with the kit may be used (e.g., Biomol). LTE4 levels are calculated by comparison to a standard curve using Microsoft Excel or other appropriate software and are expressed as ng/ml. Percent of control LTE4 release is calculated by:

% control LTE4 = (LTE4 sample-LTE4 blank)/(LTE4 total-LTE4 blank)

where LTE4 blank is the LTE4 release in the absence of Sephadex G-15 and LTE4 total is the LTE4 release in the presence of Sephadex G-15. Where applicable, an IC50 is determined for test compounds using Microsoft Excel or other appropriate software.

Evaluation of the Emetic Activity of Compounds

Five male ferrets were dosed with the test compound (either oral or i.p) then placed in individual plexiglass cages. The vehicle for oral dosing was 2% Tween 80 in 3 mL distilled water. The vehicle for i.p. dosing was 3 mL distilled water.

Animals were continuously watched for the study period (typically 60 minutes). The following behaviors were scored: (1) productive vomiting, (2) Retching (non-productive rhythmic abdominal contractions against a closed glottis). (3) Gags, where the animal strains against a closed glottis with mouth open (this did not include rhythmic abdominal contractions). For each animal, the number of each behavior and the time at which the emesis occurred was recorded. Other behaviors might be noted as well.

Ferrets were not restudied for 7 days. Studies with the PDEIV inhibitor, rolipram, indicated that ferrets cold be exposed weekly to this emetic stimulus 3 times without exhibiting emesis to saline on week 4. After 4 weekly exposures to rolipram ferrets exhibited anticipatory emesis to saline challenge on week 5. Therefore animals used in this screen were only exposed to emetic stimuli 3 times then they were removed from the colony.

If plasma drug levels were required, animals were anesthetized with Rompum and Ketamine and then 1 mL of blood was drawn from the heart using standard cardiac-puncture techniques.

Data were expressed as the number of vomits, retches and gags per group of 5 animals. The ratio of animals

exhibiting vomits, retching or gags to the total number of animals was calculated as [(# exhibiting emesis/total # in test group) x 100].

## Claims

**1.** The use of a compound of the formula I

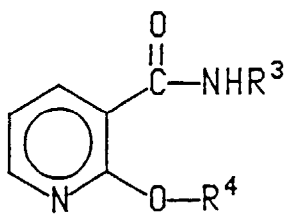

I ,

or a pharmaceutically acceptable acid addition salt thereof, wherein

$R^3$ is 1-piperidyl, 1-(3-indolyl)ethyl, $(C_1-C_4)$-alkyl, phenyl, benzyl, 1-(1-phenylethyl) or monosubstituted benzyl wherein the substituent is chloro, fluoro methyl or methoxy and said substituent is on the aromatic ring;
$R^4$ is bicyclo[2.2.1]hept-2-yl or a group of the formula II

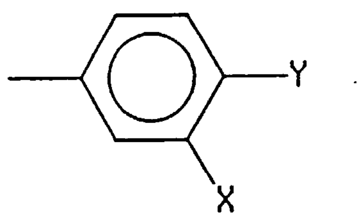

II

wherein Y is hydrogen, fluoro or chloro; and
X is hydrogen, fluoro, chloro, methoxy, trifluoromethyl, cyano, carboxy, methylcarbamoyl, dimethyl-carbamoyl or carbo$(C_1-C_4)$alkoxy;

for the manufacture of a medicament for inhibiting phosphodiesterase (PDE) type IV or the production of tumor necrosis factor (TNF).

**2.** The use according to claim 1 wherein in the compound of the formula I $R^3$ is 1-piperidyl or 1-(3-indolyl)ethyl.

**3.** The use according to claim 2 wherein in the compound of the formula I $R^3$ is 1-(3-indolyl)ethyl and $R^4$ is bicyclo [2.2.1]hept-2-yl.

**4.** The use according to either one of claims 1 and 2 wherein in the compound of the formula I $R^4$ is a group of the formula II

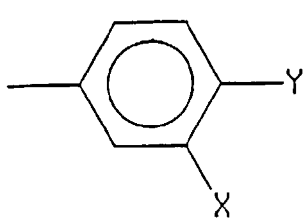

II

wherein Y is hydrogen and X is carbo$(C_1-C_4)$alkoxy.

**5.** The use according to any one of claims 1, 2 and 4 wherein in the compound of the formula I $R^4$ is 1-piperidyl and $R^4$ is a group of the formula III

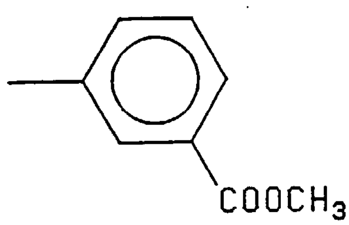

III.

**6.** The use according to any one of claims 1 to 5 wherein the medicament is for the treatment of an acute or chronic allergic or inflammatory disease selected from the group consisting of asthma, chronic bronchitis, atopic dermatitis, urticaria, allergic rhinitis, allergic conjunctivitis, vernal conjunctivitis, cosinophilic granuloma, psoriasis, rheumatoid arthritis, septic shock, ulcerative colitis, Chron's disease, reperfusion injury of the myocardium and brain, chronic glomerulonephritis, endotoxic shock, adult respiratory distress syndrome, diabetes insipidus, multiple sclerosis and central nervous system disorders, as well as other diseases involving the production of TNF.